(19) Europäisches Patentamt
European Patent Office
Office européen des brevets

(11) **EP 3 817 795 B1**

(12) **EUROPÄISCHE PATENTSCHRIFT**

(45) Veröffentlichungstag und Bekanntmachung des Hinweises auf die Patenterteilung:
**08.05.2024   Patentblatt 2024/19**

(21) Anmeldenummer: **19749175.6**

(22) Anmeldetag: **05.07.2019**

(51) Internationale Patentklassifikation (IPC):
**A61M 13/00** *(2006.01)*    **A61B 17/34** *(2006.01)*

(52) Gemeinsame Patentklassifikation (CPC):
**A61M 13/003;** A61B 17/3474; A61M 2205/15; A61M 2205/3334; A61M 2205/3344; A61M 2205/3365; A61M 2205/75

(86) Internationale Anmeldenummer:
**PCT/DE2019/000176**

(87) Internationale Veröffentlichungsnummer:
**WO 2020/007386 (09.01.2020 Gazette 2020/02)**

(54) **INSUFFLATIONSVORRICHTUNG MIT INTELLIGENTER STEUERUNG DER RAUCHGASABSAUGUNG**

INSUFFLATION DEVICE WITH INTELLIGENT CONTROL OF SMOKE EVACUATION

DISPOSITIF D'INSUFFLATION AVEC COMMANDE INTELLIGENTE DE L'ASPIRATION DE GAZ DE FUMÉE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **05.07.2018   DE 102018005314**

(43) Veröffentlichungstag der Anmeldung:
**12.05.2021   Patentblatt 2021/19**

(73) Patentinhaber: **W.O.M. World of Medicine GmbH 10587 Berlin (DE)**

(72) Erfinder: **BISCHOF, Jan 10719 Berlin (DE)**

(74) Vertreter: **Gulde & Partner Patent- und Rechtsanwaltskanzlei mbB Wallstraße 58/59 10179 Berlin (DE)**

(56) Entgegenhaltungen:
**WO-A1-2004/009167    WO-A1-2015/043570 WO-A1-2018/108200**

## Beschreibung

Die vorliegende Erfindung betrifft einen Insufflator mit integrierter Rauchgasabsaugung. Eine neuartige Steuerung der Rauchgasabsaugung ermöglicht den Druck im Patienten auch bei Leckagen aufrecht zu halten und den Absaugvolumenstrom bei hohen Leckagen zu begrenzen.

## Hintergrund und Stand der Technik

Insufflatoren mit der Möglichkeit einer gleichzeitigen Rauchgasabsaugung sind aus dem Stand der Technik bekannt (siehe z. B. WO 2015/043570 A1). Dieser Insufflator weist einen Schlauch auf, durch den ein medizinisches Gas in eine Körperhöhle (z. B. ein Abdomen) eingeleitet wird. Das Gas erzeugt einen Überdruck, welches die Körperhöhle aufdehnt, damit ausreichend Platz für die visuelle Inspektion bzw. den therapeutischen Eingriff besteht. Über einen zweiten Schlauch wird das Gas aus dem Bauchraum wieder abgesaugt. Im Falle von therapeutischen Eingriffen mittels Elektrochirurgie oder Laser können gesundheitsschädliche Rauchgase entstehen, die durch den Insufflator über diesen zweiten Schlauch abgeführt und gefiltert werden. Der Chirurg wählt einen gewünschten Soll-Absaugvolumenstrom. Die Absaugpumpe wird auf den gewünschten Soll-Absaugvolumenstrom geregelt. Problematisch ist, wenn ein größerer Leckagevolumenstrom auftritt oder der Durchflusswiderstand der Insufflationsleitung sich erhöht. In beidem Fällen ist der Insufflator nicht in der Lage die notwendige Menge $CO_2$ nach zu insufflieren. Die Folge ist das die Kavität teil oder vollständig kollabiert. Stand der Technik um diesem Effekt zu verringern, ist den Absaugvolumenstrom in solchen Situationen zu begrenzen oder zu reduzieren. Eine Möglichkeit der Umsetzung ist es zu erkennen, dass wenn sich der gemessene Druck in der Kavität von dem gewünschten Sollwert unterscheidet und daraufhin den maximalen Absaugvolumenstrom zu reduzieren.

Die meisten am Markt verfügbaren Insufflatoren arbeiten mit einem pulsartigen Verfahren um den zu insufflierenden Volumenstrom zu regeln. Hierbei wechseln sich Insufflationsphasen mit sogenannten "Messpausen" ab. In den Messpausen wird der Volumenstrom für wenige hundert Millisekunden abgestellt, so dass sich Druck im Schlauch und der Druck in der Kavität ausgleichen. Hierdurch kann der Insufflator durch einen Drucksensor in der Insufflationsleitung kurzzeitig den Druck in der Kavität messen. Über die Länge der Insufflationsphasen, wird dann der mittlere Insufflationsvolumenstrom gesteuert. Bei hohen erforderlichen Volumenströmen werden lange Insufflationsphasen durchgeführt. Bei einem solchen pulsierenden Insufflationsverfahren, besteht eine weitere Möglichkeit zur Regelung des Absaugstromes darin, die Länge einer Insufflationsphase als Kriterium für eine Verringerung oder Vergrößerung des Absaugvolumenstromes zu verwendet. Überschreitet beispielsweise die notwendige Insufflationsphasenlänge einen zuvor definierten Wert, wird die Rauchgasabsaugung reduziert.

In der Praxis hat sich gezeigt, dass diese beiden Verfahren zur Begrenzung des Absaugvolumenstromes Nachteile haben. Wichtigster Nachteil ist, dass der Druck in der Kavität sich bereits reduziert haben muss damit der Absaugvolumenstrom angepasst wird. Das bedeutet, dass es beim Öffnen von Leckagen zu unerwünschten Druckabfällen kommt, welche zu Verzögerungen der Operation führen können. Ein Beispiel hierfür ist eine größere Leckage, die während der Operation auftritt.

Moderne Insufflatoren sind in der Lage Insufflationsvolumenströme von über 30 lpm zu generieren. Hierdurch können auch hohe Leckagen ausgeglichen werden und gleichzeitig ein ausreichender Puffer für Absaugung bereitgestellt werden. Dies hat jedoch den folgenden Nachteil: Auch bei hohen Leckagen, bei welchen für Chirurgen eigentlich keine Absaugung benötigt, wird die Absaugung nicht reduziert. Die Absaugung läuft mit voller Absaugleistung weiter und belastet den Patienten durch zusätzliche Abkühlung und Austrocknung des Gewebes. Um diesen Nachteil zu verringern, wird in US-Patent US 5,199,944 ein Verfahren aufgezeigt, welches nur bei Rauchgasentstehung die Rauchgasabsaugung startet. Weiterer Stand der Technik ergibt sich aus der Druckschrift US 2018/0133416 A1. In der vorliegenden

Patentschrift wird ein alternatives Verfahren beschrieben.

Die vorliegende Erfindung wird im Anspruch 1 definiert und betrifft einen Insufflator für die minimal-invasive Chirurgie, enthaltend a) einer Druck- und Durchflussregeleinheit mit Proportionalventil, Drucksensor und Durchflussmessung b) eine Zufuhrleitung mit, optionalem Filter und Anschluss an einen ersten Trokar, c) einen zweiten Trokar mit Absaugschlauch und optionalem Filter, angeschlossen an eine Absaugvorrichtung mit regelbarer Absaugleistung, d) einer optionalen Durchflussmessung in der Absaugleitung, e) einer elektronische oder mechanischen Regeleinheit, und f) einem neuartigen Verfahren zur Einstellung des Absaugvolumenstromes.

Bei geringen Leckagen wird der Insufflator so betrieben, wie es im Stand der Technik (WO 2015/043570 A1) beschrieben ist. Der Gasanschluss (1) führt über eine Leitung (2) zur Druck- und Durchflussregeleinheit (3) der Zuführleitung (4). Im Rahmen der Druck- und Durchflusseinheit (3) ist ein Drucksensor eingerichtet, um den Druck in der Leitung zu überwachen. Außerdem ist ein Durchflussmesser (5) zur Volumenstrommessung nach der Druck- und Durchflusseinheit (3) angebracht. Ferner ist ein Filter zum Schutz des Patienten vorgesehen (6). Die Zuführleitung (7) mündet in einen ersten Trokar (8), der die Körperhöhle mit Gas befüllen kann. Der Insufflator enthält weiterhin einen zweiten Schlauch (10), der als Absaugschlauch dient. Ein zweiter in die Körperhöhle eingeführter Trokar (9) wird mittels dieses Absaugschlauches (10) mit dem Insufflator verbunden. Auch der Absaugschlauch enthält einen optionalen Filter (11) und führt über eine Leitung (13) zu einer Absaugpumpe (14). Die Absaugleistung der Absaugpumpe ist regelbar. Über eine Leitung (15) wird das Rauchgas aus dem Insufflator geführt (16). Der Absaugvolumenstrom kann entweder über den optionalen Durchflusssensor (12) gemessen werden oder über die Leistung der Absaugpumpe (14) bestimmt werden. Im Gegensatz zu Insufflatoren aus dem Stand der Technik enthält der erfindungsgemäße Insufflator eine neuartige intelligente Steuerung des Absaugvolumenstroms.

Das neuartige Verfahren zur Einstellung des Absaugvolumenstroms teilt sich in folgende Schritte:

(a) Bestimmung der momentanen maximalen Insufflationsleistung: Der maximale Insufflationsvolumenstrom ist hauptsächlich abhängig vom Durchflusswiderstand der verwendeten Trokar-Instrument-Kombination sowie dem maximalen Druck. Dieser Druck wird typischerweise auf einen Wert festlegt wird, welcher noch zu einem vertretbaren Risiko für den Patienten führt und nicht weiter erhöht werden darf. Um den maximale mögliche Insufflationsvolumenstrom zu bestimmen, können verschiedene Verfahren verwendet werden. Beispielsweise kann ein Algorithmus verwendet werden, welcher die Kennlinie der Trokar-Instrument bestimmt. Alternativ können mathematische Modelle, wie beispielsweise den Kalmanfilter oder Luenberger-Beobachter, verwendet werden um die maximale Insufflationsleistung zu bestimmen.

(b) Bestimmung der momentanen Leckagevolumenstroms $q_{Leakage}$: Auch die Leckage kann über ein mathematisches Modell geschätzt werden. Alternativ kann die mittlere Leckage auch näherungsweise durch eine Subtraktion des gemittelten Absaugungsvolumenstroms vom gemittelten Insufflationsvolumenstrom berechnet werden.

(c) Berechnung des maximalen möglichen Absaugvolumenstromes $q_{Absaug,max}$: Durch Subtraktion der bestimmten Leckage vom maximalen Insufflationsstrom und anschließender Multiplikation mit einem Sicherheitsfaktor zwischen 0 und 1, welcher definiert, mit welcher Last der Insufflator idealerweise beansprucht wird. Der Wert liegt typischerweise zwischen 0.6 und 0.9.

(d) Überprüfung ob ein maximaler Austauschdurchfluss überschritten wird:
Um zu verhindern, dass ein hoher Absaugvolumenstrom eingestellt bleibt, auch wenn in dieser Situation bereits Leckagen für einen hohen Gasaustausch sorgen, wir der Absaugvolumenstrom zusätzlich begrenzt. Hierfür wird ein maximaler Austauschvolumenstrom $q_{Austausch,max}$ definiert. Typische Werte für $q_{Austausch,max}$ liegen zwischen 10 und 20 lpm. Der Wert kann entweder über eine graphische Oberfläche angepasst werden oder ist fest im Insufflator konfiguriert oder von der Software angepasst werden. Der maximale Absaugvolumenstrom $q_{Absaug,max}$ wird dann so begrenzt, dass die Gleichung $q_{Absaug,max} + q_{Leakage} \leq q_{Austausch,max}$ erfüllt ist. In Worten. Die Summe aus maximale Absaugvolumenstrom und geschätzter Leckage darf nicht größer sein als ein definierter Wert für $q_{Austausch,max}$.

(d) Falls der vom Chirurgen eingestellte Absaugvolumenstrom größer als der berechnete maximale Absaugvolumenstrom $q_{Austausch,max}$, wird der Sollabsaugvolumenstrom auf den maximal erlaubten Absaugvolumenstrom $q_{Austausch,max}$ begrenzt.

(e) Die Berechnung wird zyklisch (beispielweise jede Sekunde) wiederholt damit der maximale erlaubte Absaugvolumenstrom sich kontinuierlich an Leckageveränderungen oder Änderungen der Trokar-Instrumenten Kombination anpassen kann.

Ein Rechenbeispiel zur Illustration:

(a) Die Kennlinie der Trokar-Instrumenten-Kombination wird zu

$$q_{instrument} = 0.2 \, \frac{lpm}{mmHg} \times p_{Schlauch}$$

mit dem Volumenstrom $q_{instrument}$ und dem Druck im Schlauch $p_{schlauch}$ bestimmt. Der sicherheitsvertretbare Druck sei 100 mmHg. Hieraus ergibt sich ein maximaler Volumenstrom von

$$q_{max} = 0.2 \, \frac{lpm}{mmHg} \times 100 \, mmHg = 20 \, lpm$$

.

(b) Die Leckage wird durch einen Beobachter oder anderes Verfahren auf 10 lpm geschätzt.

(c) Der Sicherheitsfaktor wird auf 0.8 gewählt. Hieraus ergibt sich ein maximaler Absaugstrom $q_{Absaug,max}$ = 20 *lpm* × 0.8 - 10 *lpm* = 6 *lpm*

(d) Wenn der Chirurg, einen Absaugvolumenstrom von angenommen 8 lpm gewählt hat, wird dieser reduziert auf den maximalen Absaugvolumenstrom $q_{Absaug,max}$ = 6 *lpm* um zu verhindern dass der Druck durch die gewählte Absaugung zu stark reduziert wird.

(e) Wenn der maximale Austauschvolumenstrom $q_{Austausch,max}$ auf 18 lpm konfiguriert wurde ist die Bedingung. $q_{Absaug,max} + q_{Leakage} \leq q_{Austausch,max}$ erfüllt. Es kommt somit zu keiner weiteren Reduktion des Absaugvolumenstroms durch den maximalen Austauschvolumenstrom. Wenn der maximale Austauschvolumenstrom $q_{Austausch,max}$ auf 12 lpm konfiguriert wäre, würde der Absaugvolumenstrom auf 2 lpm reduziert werden.

(f) Die Berechnung wird zyklisch wiederholt und wenn beispielweise die maximale Insufflationsleistung $q_{max}$ sich reduziert, wird auch $q_{Absaug,max}$ angepasst.

**Patentansprüche**

1. Insufflator für die minimal-invasive Chirurgie, enthaltend

   a) Gasanschluss mit Druck- und Durchflussregeleinheit ausgestattet mit Proportionalventil und Drucksensor,
   b) Zuführleitung mit optionalem Filter und Anschluss an einen ersten Trokar,
   c) zweiter Trokar mit Schlauch und optionalem Filter, angeschlossen an eine Absaugvorrichtung mit regelbarer Absaugleistung,
   d) elektronische oder mechanische Regelungseinheit,
   e) eine Vorrichtung zur Bestimmung des Absaugvolumenstromes, , wobei die Vorrichtung zur Bestimmung des Absaugvolumenstromes in Form einer Durchflussmesseinrichtung oder durch Bestimmung der Leistung der Absaugpumpe realisiert ist,

   wobei die Regelungseinheit ausgebildet ist um die Absaugleistung der Rauchgasabsaugung durch folgende Schritte in Abhängigkeit vom Bedarf zu regeln:

   a. Ein fester maximaler Insufflationsvolumenstrom $q_{max}$ wird bestimmt durch Festlegung des maximalen Druckes und Berücksichtigung des Durchflusswiderstandes der verwendeten Trokar-Instrument-Kombination, und ein maximaler Austauschvolumenstrom $q_{Austausch,max}$ wird eingestellt,
   b. Bestimmung des momentanen Leckagevolumenstroms $q_{Leakage}$ durch ein mathematisches Modell,
   c. Berechnung des maximalen möglichen Absaugvolumenstromes $q_{Absaug,max}$: durch Subtraktion der bestimmten Leckage vom maximalen Insufflationsstrom $q_{max}$ und anschließender Multiplikation mit einem Sicherheitsfaktor zwischen 0.6 und 0.9,
   d. Zyklische Überprüfung ob der Absaugvolumenstrom plus die geschätzte Leckage kleiner ist als der eingestellte maximaler Austauschvolumenstrom ($q_{Absaug,max} + q_{Leakage} < q_{Austausch,max}$),
   e. Falls die Bedingung $q_{Absaug,max} + q_{Leakage} < q_{Austausch,max}$ zutrifft wird der Absaugvolumenstrom $q_{Absaug,max}$ von der Pumpe eingestellt,

      falls die Bedingung nicht zutrifft, wird der Absaugvolumenstrom $q_{Absaug,max}$ soweit reduziert das die Bedingung erfüllt ist,
      falls $q_{Absaug,max}$ sich nicht weiter reduzieren lässt, wird $q_{Absaug,max}$ zu null gewählt

   f. Zyklische Wiederholung der Schritte zur Anpassung an Leckageveränderungen oder Änderungen der Trokar-Instrument-Kombination.

**Claims**

1. An insufflator for minimally invasive surgery, containing

   a) a gas connection having a pressure and flow rate control unit equipped with a proportional valve and a pressure sensor,
   b) a supply line having an optional filter und connection to a first trocar,
   c) a second trocar having a tube and optional filter, connected to a suction device having an adjustable suction power,

d) an electronic or mechanical control unit,

e) a device for determining the suction flow rate,

the device for determining the suction flow rate being realized in the form of a flow rate measurement unit or by determining the power of the suction pump,

the control unit being designed to control the suction power of the waste gas suction by the following steps, as required:

a. a fixed maximum insufflation flow rate $q_{max}$ is determined by defining the maximum pressure and taking into account the flow resistance of the used trocar/instrument combination, and a maximum exchange flow rate ($q_{suction,max} + q_{leakage} < q_{exchange,max}$) is set,

b. determining the current leakage flow rate $q_{leakage}$ by a mathematical model,

c. calculating the maximum possible suction flow rate $q_{suction,max}$: by subtracting the determined leakage from the maximum insufflation flow rate $q_{max}$ and subsequently multiplying the result by a safety factor of between 0.6 and 0.9,

d. cyclically checking whether the suction flow rate plus the estimated leakage is smaller than the set maximum exchange flow rate $q_{suction,max}$

e. if the condition ($q_{suction,max} + q_{leakage} < q_{exchange,max}$) is met, the suction flow rate $q_{suction,max}$ of the pump is adjusted,

if the condition is not met, the suction flow rate $q_{suction,max}$ is reduced to such an extent that the condition is met,

if $q_{suction,max}$ cannot be further reduced, a zero value is selected for $q_{suction,max}$

f. cyclically repeating the steps in order to adapt to leakage changes or changes to the trocar/instrument combination.

**Revendications**

1. Insufflateur pour la chirurgie mini-invasive, contenant

a) un raccord de gaz avec une unité de régulation de pression et de débit équipée d'une vanne proportionnelle et d'un capteur de pression,

b) une conduite d'alimentation avec un filtre optionnel et un raccord à un premier trocart,

c) un deuxième trocart avec un tuyau et un filtre optionnel, raccordé à un dispositif d'aspiration à puissance d'aspiration régulable,

d) une unité de régulation électronique ou mécanique,

e) un dispositif de détermination du débit volumétrique d'aspiration,

le dispositif de détermination du débit volumique d'aspiration étant réalisé sous la forme d'un appareil de mesure de débit ou par détermination de la puissance de la pompe d'aspiration,

l'unité de régulation étant configurée pour réguler la puissance d'aspiration de l'évacuateur de fumée en fonction des besoins par les étapes suivantes :

a. un débit volumétrique d'insufflation maximal fixe $q_{max}$ est déterminé en fixant la pression maximale et en tenant compte de la résistance à l'écoulement de la combinaison trocart-instrument utilisée, et un débit volumétrique d'échange maximal $q_{échange,max}$ est ajusté,

b. la détermination du débit volumétrique de fuite instantané $q_{fuite}$ par un modèle mathématique,

c. le calcul du débit volumétrique d'aspiration maximal possible $q_{aspiration,max}$ : en soustrayant la fuite déterminée du débit d'insufflation maximal $q_{max}$ puis en multipliant par un facteur de sécurité compris entre 0,6 et 0,9,

d. la vérification cyclique que le débit volumétrique d'aspiration plus la fuite estimée est inférieur au débit volumétrique d'échange maximal ajusté ($q_{aspiration,max} + q_{fuite} < q_{échange,max}$),

e. Si la condition ($q_{aspiration,max} + q_{fuite} < q_{échange,max}$) est vérifiée, le débit volumétrique d'aspiration $q_{aspiration,max}$ de la pompe est ajusté,

si la condition n'est pas remplie, le débit volumétrique d'aspiration $q_{aspiration,max}$ est réduit jusqu'à ce que la condition soit remplie,

si $q_{aspiration,max}$ ne peut pas être réduit davantage, $q_{aspiration,max}$ est sélectionné à zéro,

f. la répétition cyclique des étapes pour s'adapter aux changements de fuites ou aux changements de combinaison trocart-instrument.

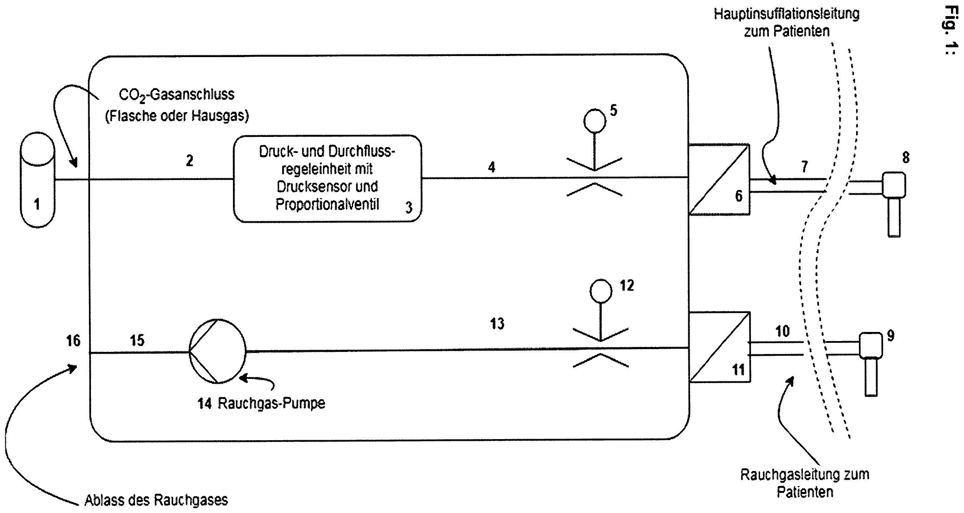

Fig. 1:

EP 3 817 795 B1

Hauptinsufflationsleitung
zum Patienten

CO₂-Gasanschluss
(Flasche oder Hausgas)

Druck- und Durchflussregeleinheit mit
Drucksensor und
Proportionalventil

14 Rauchgas-Pumpe

Ablass des Rauchgases

Rauchgasleitung zum
Patienten

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2015043570 A1
- US 5199944 A
- US 20180133416 A1